# EUROPEAN PATENT SPECIFICATION

(11) **EP 1 061 803 B1**
(45) Date of publication and mention of the grant of the patent: **11.05.2011**
(21) Application number: 99909780.1
(22) Date of filing: 11.03.1999
(51) Int. Cl.: A01N 43/50, A61K 9/14

(54) **COMPOSITIONS OF EPROSARTAN**
EPROSARTAN-ZUSAMMENSETZUNGEN
COMPOSITIONS A BASE D'EPROSARTAN

(30) Priority: 11.03.1998 US 77660 P
(43) Date of publication of application: 27.12.2000
(73) Proprietor: GlaxoSmithKline LLC, Philadelphia, PA 19102 (US)
(72) Inventor: VENKATESH, Gopadi, M., King of Prussia, PA 19406 (US)
(74) Representative: Verhage, Marinus
(86) International application number: PCT/US1999/004651
(87) International publication number: WO 1999/045779

(56) References cited:
- WO-A-97/36874
- DATABASE EMBASE ON STN, No. 95358928, MEDICINAL CHEMISTRY DEPARTMENT, INSTITUTOS LUSOFARMACO, MILANO, ITALY, SALMBENI et al., "N-3-Substituted Pyrimidinones as Potent, Orally Active, AT1 Selective Angiotensin II Receptor Antagonists"; & JOURNAL OF MEDICINAL CHEMISTRY, 1995, 38/24, pages 4806-4820.
- DATABASE TOXLIT ON STN, No. 1994:35332, DEP. RENAL PHARMACOL., SMITHKLINE BEECHAM PHARM., BROOKS et al., "Blood Pressure Lowering Activity of Enalapril and the Nonpeptide Angiotensin II Receptor Antagonist, SK&F 108566, in Furosemide- Treated Conscious Eynomolgus Monkeys"; & PHARMACOL. COMMUN., 1993, Vol. 2, No. 4, pages 331-337.

## Description

### Field of the Invention

This invention relates to novel compositions of (E)-α-[2-n-butyl-1-[(4-carboxyphenyl)methyl]-1H-imidazol-5-yl]methylene-2-thiophenepropionic acid [eprosartan] or its methanesulfonate salt [eprosartan mesylate], and to the use of such compositions in therapy to block angiotensin II (AII) receptors and in the treatment of hypertension, congestive heart failure and renal failure.

### Background of the Invention

The compound, (E)-α-[2-n-butyl-1-[(4-carboxyphenyl)methyl]-1H-imidazol-5-yl]methylene-2-thiophenepropionic, is known by the name "eprosartan" and is the subject of U.S. Patent No. 5,185,351 (the 351 patent), issued February 9, 1993. This patent discloses a process for making the anhydrous form of (E)-α-[2-n-butyl-1-[(4-carboxyphenyl)methyl]-1H-imidazol-5-yl]methylene-2-thiophenepropionic acid and its methanesulfonate salt. Additionally, the 351 patent discloses conventional techniques for formulating (E)-α-[2-n-butyl-1-[(4-carboxyphenyl)methyl]-1H-imidazol-5-yl]methylene-2-thiophenepropionic acid. Eprosartan mesylate has the following structure:

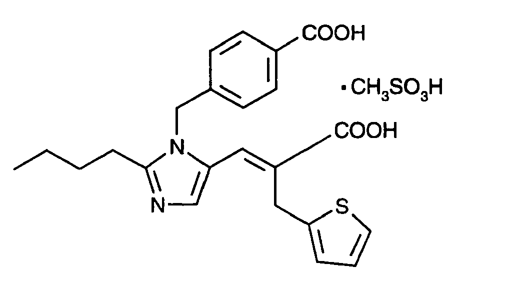

This compound is claimed to have utility in blocking angiotensin II receptors and to be useful in the treatment of hypertension, congestive heart failure and renal failure.

It is known that pharmaceutically active compounds may be subjected to milling procedures to obtain a particle size appropriate for tablet formation and for other formulation types. Air jet milling and fluid energy milling (micronising) have been favoured because of the reduced risk from introducing contamination from mill materials. However, wet milling processes have been proposed for preparation of finely divided particles for pharmaceutical use, for example see U.S. Patent No. 5,145,684. This patent discloses a wet milling procedure to produce particles of a crystalline drug substance having a surface modifier adsorbed on the surface thereof in an amount sufficient to maintain an effective average particle size of less than about 400 nm. This particulate composition as a stable suspension is said to provide improved bioavailability for poorly water soluble compounds.

WO97/36874 (SmithKline Beecham Corporation) relates to (E)-α-[2-n-butyl-1-[(4-carboxyphenyl)methyl]-1H-imidazol-5-yl]methylene-2-thiophenepropionic acid monomethanesulfonate dihydrate, a wet granulation process for preparing said compound, compositions containing this compound, and methods of using (E)-α-[2-n-butyl-1-[(4-carboxyphenyl)methyl]-1H-imidazol-5-yl]methylene-2-thiophenepropionic acid monomethanesulfonate dihydrate to block angiotensin II (AII) receptors and to treat hypertension, congestive heart failure and renal failure.

According to the instant invention, it has been found that eprosartan, or its salts, solvates, or hydrates, can be formulated in novel compositions that have a 2-5 fold enhanced therapeutic activity over that of conventionally-prepared immediate release tablet formulations of the same compound. These novel compositions have enhanced bioavailabitity.

### Summary of the Invention

The present invention is based on the finding that (E)-α-[2-n-butyl-1-[(4-carboxyphenyl)methyl]-1H-imidazol-5-yl]methylene-2-thiophenepropionic acid, or its salt forms, such as the mesylate, have enhanced bioavailability in certain compositions of controlled particle size. Anhydrous eprosartan, or its salts, or its solvates or hydrates, may be used in the compositions of the instant invention.

The present invention provides novel compositions comprising eprosartan, or a salt, solvate, or hydrate thereof, in particulate form, said composition having a particle size distribution such that the median value of the volume mean diameter is within the range of from 450 to 700 nm.

This invention also provides a process or a method of producing such compositions in a reproducible manner for the treatment of diseases in which blockade of angiotensin II receptors is indicated, for example, in the treatment of hypertension, congestive heart failure and renal failure.

### Description of the Invention

According to the present invention, compositions comprising eprosartan, or its salt, are provided in particulate form, such as immediate release (1R) capsules and immediate release/delayed release/ modified release tablets. The compositions are prepared using a process that involves a wet milling step in order to produce a particle size distribution of eprosartan, or its salt, with a volume average median diameter in the range of 450-700 nm. The particulate suspension, thus produced, is spray dried using a spray dryer or granulated using a fluid bed granulator. Compositions are prepared by admixture and, thus, they are suitably adapted for oral, parenteral or pulmonary administration. The compositions may be in the form of tablets, capsules, reconstitutable powders or suppositories. Orally administrable Orally administrable compositions are preferred, in particular shaped oral compositions, since they are more convenient for mass therapy.

To assist in further processing, that is the preparation of pharmaceutical compositions for therapeutic use, such as capsules and tablets, the wet milling of eprosartan, or its salt, takes place in an aqueous medium containing one or more pharmaceutically acceptable water-soluble carriers suitable for spray drying. The aqueous suspension may optionally contain a surfactant to maintain the particles in suspension until administration of the pharmaceutical formulation to a patient. Pharmaceutically acceptable excipients most suitable for spray-drying are water soluble hydroxypropylmethyl cellulose, a binder, and mannitol, but other binders, such as polyvinylpyrrolidone (PVP), hydroxypropyl cellulose (HPC), and methyl cellulose, or other carbohydrates, such as sucrose, sorbitol, lactose, lactitol and xylitol and starch may also be used as a carrier.

In the aqueous medium to be subjected to the milling, eprosartan, or its salt, may be present from about 10 to about 40% w/w. In practice, 20% w/w drug loading provides an effective compromise between the desire for a high throughput and short milling times.

The amount of the primary carrier, such as hydroxypropylmethyl cellulose (HPMC), may vary from about 2 to about 20% w/w of the composition to be milled. The secondary carrier, such as mannitol, may also be added to the suspension prior to milling or dissolved in the milled suspension prior to spray drying. Preferably, the total amount of the soluble carrier(s) does not exceed 100% by weight of eprosartan, or its salt, to be processed. For an eprosartan loading of about 20% w/w, an amount of HPMC from about 4 to 12% w/w has been found to be effective, and an amount of about 6% w/w is preferred.

The amount of surfactant/glidant, if present, may be varied from about 0.1 to about 0.4% w/w of the aqueous medium. Preferably, it is present at about 1 % by weight of eprosartan, or its salt. Suitable surfactants are sodium laurel sulphate and tween 80, while suitable glidents are silicon dioxide and talc.

The compositions of this invention are most suitably prepared by wet milling eprosartan, or its salt, preferably using a bead mill such as Premier HML Laboratory Supermill manufactured by Premier Mill Corporation, Reading, PA. The milling medium consists of zirconium oxide beads. Bead mills manufactured by others such as Dena Mill by Dena Systems BK Ltd., Barnsley, England, can be used for wet milling eprosartan, or its salt. The particle size distributions of the suspension formulations were determined using a Malvern laser diffraction unit, Master Sizer S Model S4700, from Malvern Instruments Ltd., Malvern, England. Any other laser diffraction particle sizer with sufficient sensitivity and resolution for nanoparticulates can be used. The particles of eprosartan, or its salt, are typically present with not less than 50% of the particles having a volume average diameter of 1000 nm or below. In a preferred embodiment of the invention, the volume averaged median diameter is in the range of 450 to 700 nm. In this median range, effective compositions are obtained when 90% of the particles have a volume averaged diameter of 1200 nm or below.

Using the milling beads and aqueous carrier system described above, a composition having the preferred particle size distribution may be obtained surprisingly quickly, for example, after milling 2.5 kg suspension in the mill for an hour. Increasing milling time, for example, by fivefold, enables the largest particles to be reduced so that at least 90% of particles have a volume average diameter of less than 1000 nm. However, the effect on the median value is marginal, so longer milling times are not cost effective.

Spray drying/fluid bed granulation of milled compositions is carried out most suitably using a spray dryer such as Yamato GA-32 Spray Dryer [Yamato Scientific America Inc., Orangeburg, NY], or a fluid bed granulator, such as Glatt fluid bed granulator.

Tablets and capsules for oral administration are usually presented in a unit dose, and contain conventional excipients such as binding agents, fillers and diluents (tableting or compression aids), lubricants, disintegrants, colorants, flavourings, and wetting agents. The tablets may be coated according to techniques well known in the art.

Suitable binders include polyvinlpyrrolidone (PVP), hydroxypropylmethyl cellulose and pregelatinized starch (Starch 1551). Suitable fillers include microcrystalline cellulose, mannitol, lactose and other similar agents. Suitable disintegrants include super disintegrants such as cross linked polyvinylpyrrolidone (Crospovidone XL), sodium starch glycolate (Explotab) and croscarmellose sodium (Ac-Di-Sol). Suitable lubricants include stearic acid and magnesium stearate.

These solid oral compositions may be prepared by conventional methods of blending, filling, tableting, or the like. Repeated blending operations may be used to distribute the active agent throughout those compositions employing large quantities of fillers. Such operations are, of course, well known in the art.

Oral formulations also include conventional controlled release formulations, such as tablets or pellets, beads or granules, having a sustained release or an enteric coating, or otherwise modified to control the release of the active compound, for example by the inclusion of gel forming polymers or matrix forming waxes.

Advantageously, a surfactant or wetting agent may be included in the composition to facilitate uniform distribution of the compound of the invention.

Thus, the present invention provides a novel composition which comprises eprosartan, or its salts, solvates, or hydrates. The composition is adapted for oral administration. The composition is presented as a unit dose. Such a composition is taken preferably from 1 to 2 times daily. The preferred unit dosage forms include tablets or capsules. The compositions of this invention may be formulated by conventional methods of admixture such as blending, filling and compressing. Suitable pharmaceutically acceptable carriers for use in this invention include diluents, fillers, binders and disintegrants.

Eprosartan, or its salts, solvates, or hydrates, may be co-administered with other pharmaceutically active compounds, for example, in physical combination or by sequential administration. Conveniently, the compound of this invention and the other active compound are formulated in a pharmaceutical composition. Thus, this invention also relates to pharmaceutical compositions comprising eprosartan, or its salts, solvates; or hydrates, a pharmaceutically acceptable carrier, and a second pharmaceutically active compound selected from the group consisting of a diuretic, a calcium channel blocker, a ß-adrenoceptor blocker, a renin inhibitor, and an angiotensin converting enzyme inhibitor. Examples of compounds which may be included in pharmaceutical compositions in combination with eprosartan, or its salts, solvates, or hydrates, are diuretics, particularly a thiazide diuretic, such as hydrochlorothiazide, or a loop diuretic, such as furosemide, calcium channel blockers, particularly dihydropyridine antagonists, such as nifedipine, ß-adrenoceptor blockers, such as propranolol, renin inhibitors, such as enalkinen, and angiotensin converting enzyme inhibitors, such as captopril or enalapril.

No unacceptable toxicological effects are expected when eprosartan, or its salts, solvates, or hydrates is administered in accordance with the present invention.

Eprosartan, or its salts, solvates, or hydrates is useful for treating diseases in which blockade of the angiotensin II receptor would be beneficial. Preferably, this compound is used alone or in combination with said second pharmaceutically active compounds in the treatment of hypertension, congestive heart failure and renal failure. Additionally, eprosartan, or its salts, solvates, or hydrates, is of value in the treatment of left ventricular hypertrophy regression, diabetic nephropathy, diabetic retinopathy, mascular degeneration, haemorrhagic stroke, primary and secondary prevention of infarction, prevention of atheroma progression and the regression of atheroma, prevention of restinosis after angioplasty or bypass surgery, improving cognitive function, angina, glaucoma, and CNS disorders, such as anxiety.

The following examples are illustrative of the instant invention.

### Examples of Nanoparticulate Suspensions

Suspensions (refer to Table 1 for formulation details) were prepared by dispersing hydroxypropylmethylcellulose (HPMC) in purified water, using a suitable mixer and stored overnight to hydrate. The drug substance was dispersed using a homogenizer and mixing continued until no drug agglomerates remained. The Premier bead mill was set up with 0.6-0.8 mm zirconium oxide beads filling about 80% of the milling chamber. The bead mill was operated in accordance with the manufacturer's instructions at the settings listed in Table 1.

After milling, the particle size distribution of the suspensions of Formulas 1-3 was measured using the Malvern laser diffraction unit. The sample was appropriately diluted, and the particle sizer was operated in accordance with the manufacturer's instructions at the settings listed in Table 1. The results are reported in Table 1 as D(0.1), D(0.5), and D(0.9). D(0.1) represents the size (as number average diameter) below which 10% of the particles lie, D(0.5) is the size below which 50% of the particles lie, also known as the median. D(0.9) is the measurement below which 90% of the particles lie.

### Examples of Bioenhanced Oral Solid Formulations

The milled suspension was spray dried using a laboratory scale Yamato GA-32 Spray Dryer. The spray dryer was operated in accordance with the manufacturer's instructions at the settings listed in Table 1. In this apparatus, the milled aqueous suspension was fed by a peristaltic pump into the drying chamber as an atomized spray. The resulting spray dried powder was further dried [for example, oven drying at about 40°C] to produce a dry powder composition which has good flowability/ compactibility properties and hence, is suitable for filling into hard gelatin capsules (Formulas 3 - 5 in Table 1).

The milled suspension was spray granulated in a fluid bed granulator. The fluid bed contained the excipients listed in Table 2 (Formulas 6 and 7). These granulations were blended with a disintegrant and a lubricant and compressed into pharmaceutically elegant tablets.

The spray dried material was further wet granulated using a high shear granulator and compressed into tablets by incorporating widely used excipients (Formula 8 in Table 2).

### Bio-Equivalence Studies

Test dogs were administered orally in a four-arm study using a dose of 10 mg of eprosartan as zwitterion per kg. The internal granules of the current product (refer to Table 3 for formulation details) was used as the control.

Bio-equivalence studies to determine the relative bioavailability of oral bioenhanced formulations in healthy volunteers were performed. The study design comprised an open, randomised, and crossover. A single oral 200 mg dose of the following formulations - a commercial tablet, a capsule of Formula 6 and a tablet of Formula 7. On each of the dosing days subjects were to receive a single oral dose, with a washout of at least 6 days between doses. Doses were administered orally with 200 mL water, following a standard breakfast. The blood samples were collected at predetermined time points and tested.

From the plasma concentration profiles obtained from the clinical studies both in dogs and humans, AUCₘₐₓ (mg.hr/ml) [area under the plasma concentration vs. time curve], Cₘₐₓ (mg/ml) [maximum plasma concentration] and tₘₐₓ (time in hours to achieve maximum plasma level) were calculated. The results, subjected to appropriate statistical tests, showed an increase in blood levels by 2-5 fold, when dosing with capsules/tablets containing the bioenhanced formulations.

**Table 1 : Bioenhanced Suspension Formulations**

| **Ingredients** | **Nanoparticulate suspension formulations- %w/w** | | | | |
|---|---|---|---|---|---|
| Formula # | 1 | 2 | 3 | 4 | 5 |
| Eprosartan mesylate | 10 | 20 | 15.7 | 14.8 | 14.4 |
| HPM cellulose | 10 | 10 | 5.9 | 5.5 | 1.5 |
| Mannitol | | | | 5.5 | 6.2 |
| Sodium lauryl sulfate | | | | | |
| Purified water | to | to | to | to | to |
| (ml) | 100 | 100 | 100 | 100 | 100 |
| | | | | | |

| **Parameter** | **Bead milling conditions** | | | | |
|---|---|---|---|---|---|
| Bead size, mm | 0.6-0.8 | 0.6-0.8 | 0.6-0.8 | 0.6-0.8 | 0.6-0.8 |
| # passes | 2 | 5 | 5 | 10 | 10 |
| | | | | | |

| **Parameter** | **Malvern Particle size Distribution** | | | | |
|---|---|---|---|---|---|
| D(0.1) in nm | 181 | 123 | 117 | 98 | 101 |
| D(0.5) in nm | 759 | 671 | 643 | 497 | 469 |
| D(0.9) in nm | 1200 | 1050 | 1097 | 893 | 879 |
| | | | | | |

| **Parameter** | **Spray drying conditions** | | | | |
|---|---|---|---|---|---|
| Inlet temp.(°C) | | | 125 | 125 | 125 |
| Outlet temp.(C) | | | 76 | 78-84 | 78-84 |
| Air flowrate (m3/min) | | | 0.35 | 0.45 | 0.45 |
| Atom. press (kg/cm2) | | | | 1.5 | 1.5 |
| Feed rate (ml/min) | | | | 5 | 5 |
| | | | | | |

**Table 2 : Bioenhanced Tablet Formulations**

| **Ingredients** | **mg/capsule or mg/tablet** | | |
|---|---|---|---|
| **Formula** | **6-Capsule** | **7-Fluid bed** | **8-High shear** |
| **Bead milling Susp.** | | | |
| Eprosartan mesylate | 245.27 | 245.27 | 245.27 |
| HPMC (Methocel E5) NF | 91.98 | 91.98 | 91.98 |
| Silicon dioxide | 5.01 | 5.01 | 5.01 |
| Purified water USP | * | * | * |
| **Fluid bed bulking** | | | ** |
| Impalpable Lactose | 57.74 | 47.74 | 47.74 |
| Microcrystalline cellulose | | 26.01 | 26 |
| Crosslinked PVP USP | | 8 | 8 |
| **Extermals** | | | |
| Crosslinked PVP USP | | | |
| Magnesium stearate | | | |

| | | | |
|---|---|---|---|
| * Water is eliminated during spray drying | | | |

**Table 3 : Formula of Eprosartan Commercial Product**

| **Ingredients** | | **% w/w** |
|---|---|---|
| **Intragranular** | | |
| Eprosartan mesylate (400 mg as zwitterion) | | 61.32 |
| Lactose, Monohydrate (Impalpable) NF | 3.59 | |
| Microcrystalline cellulose (Avicel PH102) NF | 3.59 | |
| Pregelatinized starch (Starch 1551) USP | 3.59 | |
| Purified water USP | | 4.36* |
| **Extragranular** | | |
| Croscarmellose sodium (Ac-Di-Sol) | | 4.00 |
| Microcrystalline cellulose (Avicel PH102) NF | 19. | |
| Magnesium stearate NF | 0.75 | |
| Tablet core weight (200 mg) | | 400.00 |
| **Film coating** | | |
| Opadry Blue OY-S-20900** | | |

| | | |
|---|---|---|
| a A control tablet formulation was produced by a high shear Fielder granulation and the weight average median diameter of the drug substance (eprosartan mesylate) was - 15 microns. * Purified water is added during granulation to form the dihydrate of the salt. ** Film coat is applied to a level of approximately 2.5-4% of core weight. | | |

## Claims

1. A pharmaceutical composition comprising eprosartan, or a salt, solvate, or hydrate thereof, in particulate form, said composition having a particle size distribution such that the median value of the volume mean diameter is within the range of from 450 to 700 nm.

2. The composition according to claim 1 wherein eprosartan, or a salt, solvate, or hydrate thereof, is eprosartan, mesylate.

3. The composition according to claim 1 which is produced by wet milling eprosartan, or a salt, solvate, or hydrate thereof, in the presence of water and pharmaceutically acceptable excipients.

4. The composition according to claim 3 wherein the excipient comprises hydroxypropylmethyl cellulose or mannitol or a mixture thereof.

5. A process for the preparation of the composition according to claim 1 in which the eprosartan, or a salt, solvate, or hydrate thereof, in particulate form, is produced by milling eprosartan, or a salt, solvate, or hydrate thereof, using a bead mill in the presence of hard zirconium oxide beads, water and hydroxypropylmethyl cellulose, optionally adding one or more pharmaceutically acceptable excipients, spray drying and filling the resulting powder into capsules.

6. The use of a composition according to claims I to 4 for the manufacture of a medicament for the treatment of diseases in which blockade of the angiotensin II receptor is indicated.

7. The use of a composition according to claims 1 to 4 for the manufacture of a medicament for the treatment of hypertension.

8. The use of a composition according to claims 1 to 4 for the manufacture of a medicament for the treatment of congestive heart failure.

9. The use of a composition according to claims 1 to 4 for the manufacture of a medicament for the treatment of renal failure.

10. The use of a composition according to claims 1 to 4 in combination with a diuretic for the manufacture of a medicament for the treatment of hypertension.

11. The use according to claim 10 wherein the diuretic is hydrochlorothiazide.

12. A composition according to claims 1 to 4 for use in the treatment of diseases in which blockade of the angiotensin II receptor is indicated.

13. A composition according to claims 1 to 4 for use in the treatment of hypertension.

14. A composition according to claims 1 to 4 for use in the treatment of congestive heart failure.

15. A composition according to claims 1 to 4 for use in the treatment of renal failure.

16. A composition according to claims 1 to 4 in combination with a diuretic for use in the treatment of hypertension.

17. A composition for use in the treatment of hypertension according to claim 16 wherein the diuretic is hydrochlorothiazide.

## Patentansprüche

1. Pharmazeutische Zusammensetzung, welche Eprosartan oder ein Salz, Solvat oder Hydrat davon in partikulärer Form umfasst, wobei besagte Zusammensetzung eine Partikelgrößenverteilung hat, so dass der Medianwert des mittleren Volumendurchmessers innerhalb des Bereichs von 450 bis 700 nm ist.

2. Zusammensetzung gemäß Anspruch 1, worin Eprosartan oder ein Salz, Solvat oder Hydrat davon Eprosartan-Mesylat ist.

3. Zusammensetzung gemäß Anspruch 1, welche hergestellt ist durch Nassmahlen von Eprosartan oder einem Salz, Solvat oder Hydrat davon, in Anwesenheit von Wasser und pharmazeutisch annehmbaren Arzneimittelträgern.

4. Zusammensetzung gemäß Anspruch 3, worin der Arzneimittelträger Hydroxypropylmethylcellulose oder Mannitol oder ein Gemisch daraus umfasst.

5. Verfahren zur Herstellung der Zusammensetzung gemäß Anspruch 1, wobei das Eprosartan oder ein Salz, Solvat oder Hydrat davon in partikulärer Form durch Mahlen von Eprosartan oder einem Salz, Solvat oder Hydrat davon unter Verwendung einer Perlmühle in Anwesenheit von harten Zirconiumoxid-Perlen, Wasser und Hydroxypropylmethylcellulose gegebenenfalls unter Zugabe von einem oder mehreren pharmazeutisch annehmbaren Arzneimittelträgern, Sprühtrocknen und Füllen des sich ergebenden Pulvers in Kapseln hergestellt wird.

6. Verwendung einer Zusammensetzung gemäß Ansprüchen 1 bis 4 für die Herstellung eines Medikaments zur Behandlung von Krankheiten, bei welchen Blockade des Angiotensin-II-Rezeptors indiziert ist.

7. Verwendung einer Zusammensetzung gemäß Ansprüchen 1 bis 4 für die Herstellung eines Medikaments zur Behandlung von Bluthochdruck.

8. Verwendung einer Zusammensetzung gemäß Ansprüchen 1 bis 4 für die Herstellung eines Medikaments zur Behandlung von kongestivem Herzversagen.

9. Verwendung einer Zusammensetzung gemäß Ansprüchen 1 bis 4 für die Herstellung eines Medikaments zur Behandlung von Nierenversagen.

10. Verwendung einer Zusammensetzung gemäß Ansprüchen 1 bis 4 in Kombination mit einem Diuretikum für die Herstellung eines Medikaments zur Behandlung von Bluthochdruck.

11. Verwendung gemäß Anspruch 10, wobei das Diuretikum Hydrochlorthiazid ist.

12. Zusammensetzung gemäß Ansprüchen 1 bis 4 zur Verwendung bei der Behandlung von Krankheiten, bei welchen Blockade des Angiotensin-II-Rezeptors indiziert ist.

13. Zusammensetzung gemäß Ansprüchen 1 bis 4 zur Verwendung bei der Behandlung von Bluthochdruck.

14. Zusammensetzung gemäß Ansprüchen 1 bis 4 zur Verwendung bei der Behandlung von kongestivem Herzversagen.

15. Zusammensetzung gemäß Ansprüchen 1 bis 4 zur Verwendung bei der Behandlung von Nierenversagen.

16. Zusammensetzung gemäß Ansprüchen 1 bis 4 in Kombination mit einem Diuretikum zur Verwendung bei der Behandlung von Bluthochdruck.

17. Zusammensetzung zur Verwendung bei der Behandlung von Bluthochdruck gemäß Anspruch 16, wobei das Diuretikum Hydrochlorthiazid ist.

## Revendications

1. Composition pharmaceutique comprenant de l'éprosartan ou un sel, solvate ou hydrate de celui-ci, sous forme particulaire, ladite composition ayant une distribution de dimension particulaire telle que la valeur médiane du diamètre moyen en volume est comprise dans la fourchette de 450 à 700 nm.

2. Composition selon la revendication 1 où l'éprosartan ou un sel, solvate ou hydrate de celui-ci est le mésylate d'éprosartan.

3. Composition selon la revendication 1 laquelle est produite par broyage au mouillé d'éprosartan ou d'un sel, solvate ou hydrate de celui-ci, en présence d'eau et d'excipients pharmaceutiquement acceptables.

4. Composition selon la revendication 3 où l'excipient comprend de l'hydroxypropylméthylcellulose ou du mannitol ou un mélange de ceux-ci.

5. Procédé de préparation de la composition selon la revendication 1 dans lequel l'éprosartan ou un sel, solvate ou hydrate de celui-ci, sous forme particulaire, est produit par broyage d'éprosartan ou d'un sel, solvate ou hydrate de celui-ci, au moyen d'un broyeur à billes en présence de billes dures d'oxyde de zirconium, d'eau et d'hydroxypropylméthylcellulose, par addition facultative d'un ou plusieurs excipients pharmaceutiquement acceptables, par séchage par pulvérisation et par introduction de la poudre obtenue dans des capsules.

6. Utilisation d'une composition selon les revendications 1 à 4 pour la préparation d'un médicament destiné au traitement de pathologies dans lesquelles est indiqué un blocage du récepteur de l'angiotensine II.

7. Utilisation d'une composition selon les revendications 1 à 4 pour la préparation d'un médicament destiné au traitement de l'hypertension.

8. Utilisation d'une composition selon les revendications 1 à 4 pour la préparation d'un médicament destiné au traitement de l'insuffisance cardiaque congestive.

9. Utilisation d'une composition selon les revendications 1 à 4 pour la préparation d'un médicament destiné au traitement de l'insuffisance rénale.

10. Utilisation d'une composition selon les revendications 1 à 4 en association avec un diurétique pour la préparation d'un médicament destiné au traitement de l'hypertension.

11. Utilisation selon la revendication 10 où le diurétique est l'hydrochlorothiazide.

12. Composition selon les revendications 1 à 4 pour utilisation dans le traitement de pathologies dans lesquelles est indiqué un blocage du récepteur de l'angiotensine II.

13. Composition selon les revendications 1 à 4 pour utilisation dans le traitement de l'hypertension.

14. Composition selon les revendications 1 à 4 pour utilisation dans le traitement de l'insuffisance cardiaque congestive.

15. Composition selon les revendications 1 à 4 pour utilisation dans le traitement de l'insuffisance rénale.

16. Composition selon les revendications 1 à 4 en association avec un diurétique pour utilisation dans le traitement de l'hypertension.

17. Composition pour utilisation dans le traitement de l'hypertension selon la revendication 16 où le diurétique est l'hydrochlorothiazide.
